# EUROPEAN PATENT SPECIFICATION

(11) **EP 2 620 768 B1**
(45) Date of publication and mention of the grant of the patent: **10.12.2014**
(21) Application number: 12000446.0
(22) Date of filing: 25.01.2012
(51) Int. Cl.: G01N 33/497

(54) **Portable sensing device**
Tragbare Detektionsvorrichtung
Dispositif de détection portable

(43) Date of publication of application: 31.07.2013
(60) Divisional application: 14002594.1
(73) Proprietor: Sensirion AG, 8712 Stäfa (CH)
(72) Inventor: Mayer, Felix, 8712 Stäfa (CH); Graf, Markus, 8005 Zürich (CH); Bürgi, Lukas, 8049 Zürich (CH); Böni, Dominic, 8157 Dielsdorf (CH)
(74) Representative: Toleti, Martin

(56) References cited:
- WO-A2-02/22007
- CH-A5- 691 197
- KR-B1- 100 690 638
- US-A1- 2005 009 195
- US-B1- 7 928 394
- STEFANO ZAMPOLLI ET AL: "Ultra-low-power components for an RFID Tag with physical and chemical sensors", MICROSYSTEM TECHNOLOGIES ; MICRO AND NANOSYSTEMS INFORMATION STORAGE AND PROCESSING SYSTEMS, SPRINGER, BERLIN, DE, vol. 14, no. 4-5, 11 October 2007 (2007-10-11), pages 581-588, XP019591714, ISSN: 1432-1858
- ANDREAS KOLL: "Flip-chip packaged CMOS chemical microsystem for detection of volatile organic compounds", PROCEEDINGS OF SPIE, vol. 3328, 1 January 1998 (1998-01-01), pages 223-232, XP55024041, ISSN: 0277-786X, DOI: 10.1117/12.320173
- NAGEL D J: "Microsensor clusters", MICROELECTRONICS JOURNAL, MACKINTOSH PUBLICATIONS LTD. LUTON, GB, vol. 33, no. 1-2, 2 January 2002 (2002-01-02), pages 107-119, XP004329611, ISSN: 0026-2692, DOI: 10.1016/S0026-2692(01)00110-0

## Description

### Technical Field

The present invention relates to a portable electronic device.

### Background Art

The more sensors are integrated into smart phones or tablet computers, for example, the more information can be gathered the user may make use of.

In KR 100 690 638 B1, a gas sensor module of a mobile terminal is provided to measure accurate alcohol concentration, to minimize space restriction on installation of internal components of a mobile terminal and to enhance the utility of space. A terminal case includes a formation hole through which external air passes. A hermetically closed case is installed within the terminal case and has gas passing holes connected with the formation hole. A gas sensor is provided within the hermetically closed case, senses a particular component of a gas introduced through the gas passing holes, and outputs a corresponding electrical signal. A flexible printed circuit board is installed to be electrically connected with the gas sensor within the terminal case in order to transfer the electrical signal outputted from the gas sensor to a main PCB.

### Disclosure of the Invention

The problem to be solved by the present invention is therefore to provide a portable electronic device for identifying selected properties of its environment.

This problem is solved by a portable electronic device according to the features of claim 1. The portable electronic device comprises a chemical sensor adapted to measure a property of different analytes. The chemical sensor is arranged inside a housing of the portable electronic device. An opening in the housing is provided for exposing the chemical sensor to a fluid to be analyzed, wherein the opening is an opening in the housing also used for arranging a speaker. Such fluid may be a fluid in an environment of the portable electronic device. In this context, a fluid may at least comprise a liquid or a gas. Such fluid may contain one or more ingredients. The detection of chemical substances also denoted as analytes contained in such fluid may be of interest to a user. Analytes may be, for example, CO2, NOX, Ethanol, ethanol, CO, ozone, ammonia, formaldehyde, or xylene without limitation. The chemical sensor is adapted to detect at least one property of at least two different analytes. Hence, a fluid supplied to the chemical sensor may be analyzed by means of the chemical sensor as to if and which of the chemical substances the chemical sensor is sensitive to are present in the fluid supplied. It is always subject to a design of the chemical sensor as to how many different analytes > 1 and/or how many different properties of an analyte the chemical sensor is receptive to. Typically, such chemical sensor is capable of detecting / measuring a property of the analytes assigned, which property may, for example, be a concentration of an analyte in a sample which sample specifically may be air surrounding the device. Other properties may be, for example, chemical properties such as a binding energy of an analyte. Or, the chemical sensor may comprise at least one receptor of a sensor material, e.g. in form of a layer, to which an analyte may bond to and as such modify an electrical property of the sensor material such as its electrical conductance, which principle preferably is applied in metal oxide chemical sensors, or an optical property of the sensor material such as its transmission rate, for example. Then, the electrical or optical property of a combination of the analyte and the receptor is measured and allows a conclusion as to the analyte, such as by way of comparison to a property of the receptor measured without the presence of the analyte. It is noted that for the different analytes - which may be different chemical elements or chemical compounds - it is not required to always measure the same property per analyte. Different properties may be measured for different analytes.

Specifically, the chemical sensor may be a gas sensor for detecting multiple substances in a gas.

The portable electronic device according to the present invention comprises a housing, and an opening in the housing. For the reason, that the chemical sensor is arranged within the housing, there is an access required for the fluid or at least analytes of the fluid to access the chemical sensor inside the housing. Such access in form of an opening in the housing may, for example, be of small scale, such as, for example, of a diameter of between 0.1 mm and 3 mm. By means of the opening in the housing the chemical sensor is sufficiently exposed to the environment for providing reasonable measurements as to the analytes the sensor is prone to. On the other hand, the chemical sensor is sufficiently protected from contamination which in turn supports an accurate and stable performance. In a majority of cases, the fluid may be a gas, and especially be the air surrounding the portable electronic device. Hence, in a sample application it may be of interest to identify if such air may contain analytes the chemical sensor is prone to. Specific applications may include the detection of toxic gases, the detection of ethanol in a users breath, or the detection of other substances. The opening may be arranged in a front wall, a bottom wall, or a side wall of the housing, and, for example, may take the form of a cavity. An arrangement in a cavity of the housing shall be included in the term "inside" the housing.

In a preferred embodiment, the portable electronic device further comprises a conductor board, which specifically may be a flexible conductor board, and in a further embodiment may be a flexible printed circuit board. In another variant a processing unit is connected to the chemical sensor via the conductor board. The processing unit may be any microprocessor present in the device, since many portable electronic devices such as smart phones or tablet computers require considerable computing capability. While the processing unit not necessarily needs to be arranged on the conductor board and may be arranged elsewhere, it is preferred that the chemical sensor not only is electrically connected to the conductor board but also is arranged on or attached to the conductor board. Hence, the conductor board may form a support for the chemical sensor such that the chemical sensor is mechanically and electrically coupled to the conductor board.

Hence, any portable electronic device such as a mobile phone, and in particular a smart phone, a handheld computer, an electronic reader, a tablet computer, a game controller, a pointing device, a photo or a video camera, or a computer peripheral - which listing is not limited - may in addition to its original function provide chemical information as to its environment. The user may learn about chemical substances and compositions present in the devices surroundings, and may use, transmit or else further analyse such information. For the reason that such portable electronic device typically includes interfaces to a remote infrastructure, such information may be transmitted elsewhere and used elsewhere. In an alternative, the user himself/herself may benefit from the information provided by the chemical sensor in that actions can be taken in response to detected analytes, including but not limited to analytes representing toxic substances. Such portable electronic device as a result may primarily be designed for computing and/or telecommunication and/or other tasks in the IT arena, and now may be enhanced by the function of providing chemical information as to its environment.

The chemical sensor not only is capable of measuring a single analyte, but instead is designed for measuring one or more properties of multiple different analytes. Hence, the chemical sensor may be understood as a sensor device for detecting one or even more properties of more than one analyte. Specifically, the sensor may for this purpose be embodied as a sensor array. In such sensor array, each sensor cell may provide a layer of a material exhibiting different absorption characteristic such that each cell of the sensor array may specifically be sensitive to a different analyte and as such may enable the portable electronic device to detect the presence or absence or concentration of such analyte. In other variants, each sensor cell may provide a sensor material, e.g. in form of a layer, and also denoted as receptor, to which an analyte may bond to and as such modify an electrical property of the sensor material such as its electrical conductance, which principle preferably is applied in metal oxide chemical sensors, or an optical property such as its transmission rate, for example. However, a sensor cell of such sensor array may in one embodiment exhibit not only sensitivity to a core analyte, but also to analytes other than the core analyte since such sensor cell may exhibit a cross-sensitivity to one or more analytes possibly representing core analytes for other cells. In another embodiment, the chemical sensor may be a single sensor cell, e.g. with a single layer, which however, may be receptive to multiple different analytes. Such single cell may, in one embodiment, be receptive to different analytes only under different operating conditions. For example, the sensor cell may be receptive to a first analyte x when being heated to a first temperature tx, and may be receptive to a second analyte y when being heated to a second temperature ty which is different from the first temperature tx. In another variant, a sensor array may comprise multiple sensor cells wherein at least one of the multiple sensor cells - and in another variant preferably all of the multiple sensor cells - is designed such that such cell/s may be sensitive to different analytes under different operating conditions such as under different temperatures. In such specific embodiment, each of such cell/s may be provided with an individual heater.

However, the chemical sensor may be based on one of the following measurement principles without limitation:
A chemomechanical principle, in which a chemical reaction is transformed into a surface acoustic wave, or into a cantilever resonance, for example. Alternatively, there may be thermal sensing concepts applied, e.g. by making use of pellistors which may serve as a catalytic thermal sensor in which heat is generated during combustion. Alternatively, the chemical sensor may rely on optical detection, such as in form of a microspectrometer, or an NDIR, or may make use of electrochemical reactions such as being enabled by solid state electrolytes in combination with voltammetric, potentiometric, or conductometric measurement principles. Chemiresistors may also be used, such as conducting and carbon-loaded polymers, preferably in a low-temperature arena, or, metal-oxide sensors such as tin oxide, gallium oxide, indium oxide, zinc oxide, which preferably may be applied in a high-temperature arena. ISFET (ion-selective FET) may also be used, as well as chemocapacitors wherein it is preferred to use a polymer as active material. In a different embodiment, metal-oxide sensors may be applied as chemical sensors.

Given that in a preferred embodiment the chemical sensor is arranged on an electrical conductor board such as a circuit board, the chemical sensor may be arranged between the conductor board and a wall of the housing which wall comprises the opening. Preferably the chemical sensor faces the opening and is located underneath the opening. In such scenario, the chemical sensor may be sandwiched between the conductor board and the wall of the housing with the opening. This improves a support for the chemical sensor and enhances the fixture of the sensor device within the housing. Still, the chemical sensor may be arranged close to the opening such that the chemical sensor has a quick response time and such that a sufficient amount of gas may be forwarded to the chemical sensor.

The conductor board may be embodied as a printed circuit board, i.e. a relatively stiff circuit board. In a different embodiment, the conductor board may be embodied as a flexible printed circuit board which is flexible and can be bent in order to follow a shape of the housing or its interior, for example.

In case of a sensor array the individual sensor cells may be embodied as discrete sensor cells arranged on the conductor board. In a different embodiment, the sensor cells may be represented by multiple chips the sensing structures are integrated in. Here, each individual chip may be packaged, i.e. encapsulated, and arranged on the conductor board. In an alternative arrangement, such multiple sensor chips may comprise a common package, such that these chips are encapsulated by a common encapsulation, which package finally is arranged on the conductor board. In a further embodiment, the sensor cells are monolithically integrated into a common sensor chip with a common substrate for all sensor cells. Such monolithic sensor chip may still be encapsulated and be arranged on and electrically connected to the conductor board.

In the arrangement illustrated above, the chemical sensor may be fixed in its position - which preferably may be a position directly underneath the opening in the housing - by a clamp mechanism in which two housing halves may be clamped together with at least the conductor board and the chemical sensor arranged in between. Preferably, a support element is arranged between a lower half of the housing and the conductor board such that this elastic element will help to have the chemical sensor be pressed against the upper housing half. For this purpose, the support element may be of elastic nature in one embodiment.

In such scenario, but also in other scenarios, it may be beneficial that a seal is arranged between the chemical sensor and the housing for sealing the opening against an interior of the housing. Such seal may be applied on top of the chemical sensor, and, for example, be glued thereto or otherwise attached thereto, however, without covering the sensitive structure of the chemical sensor, but preferably encircling the sensitive structure instead. On top of the chemical sensor may include on top of the sensor chip, or on top of an encapsulation of the sensor chip. When clamping the housing together, the seal preferably is arranged such that it encloses the opening in the housing. Alternatively, the seal may be glued or otherwise attached to the housing and specifically to its inner wall thereby enclosing the opening in the housing wall such that when clamping the housing together, the seal preferably rests on the chemical sensor and encloses the sensitive structure. In a third alternative, the seal is placed between the chemical sensor and the housing without being attached to any of these elements and is held between these elements when the housing halves are clamped together. In all variants, the seal may take the form of a seal ring, i.e. a closed structure, and be of a photoresist such as SU8, or epoxy material, or a material with elastic properties, for example. In other variants, a sealing compound may be applied between the chemical sensor and the housing where needed. After being hardened such sealing compound provides for the sealing function.

In another embodiment, a seal ring may be arranged between the conductor board and the wall of the housing comprising the opening. The seal may be of an arbitrary ring structure and be made of an appropriate material. It may enclose the chemical sensor. When clamping the two housing halves together, the seal, and if present, the support element may be compressed thereby improving the sealing function.

The seal may be preferred not only for protecting the inside of the portable electronic device, but also improving the chemical measurements, the response time in particular: For the reason that the seal prevents any gas from entering - by diffusion, for example - into the housing through the assigned opening, no transient sensor states, where the gas concentration in dead volumes of the housing are equilibrated by gas exchange processes through the assigned opening among others, can occur. In such transient states the gas concentration the sensor is exposed to is governed by the exact nature of the exchange process through the assigned opening and lies in between the gas concentration in the surrounding and the one in the dead volumes of the mobile device.

In a different embodiment, the chemical sensor may not be arranged underneath the opening in the housing with its sensitive structure facing the opening. Here, a side wall of the housing, instead of one of the top and bottom walls, may comprise the opening while the chemical sensor still is arranged on a conductor board orthogonal to such side wall.

In yet another alternative, the conductor board may be arranged in between the chemical sensor and the wall of the housing comprising the opening. Hence, the chemical sensor is arranged on the back side of the conductor board. In this scenario, the chemical sensor chip may be flip chip mounted to the conductor board such that the sensitive element faces the conductor board. In order to now expose such chemical sensor sufficiently to the environment, the conductor board may have an opening for allowing the fluid from the outside to reach the chemical sensor.

The opening in the housing may, in a preferred embodiment, be covered by a membrane at least permeable to analytes of interest or permeable to the gas of interest, by a grid, by slits or by a bezel. By such means, the chemical sensor and the interior of the housing may be protected.

Instead of providing a single opening in the housing there may be provided two or more openings in the housing, two of which openings may be arranged on opposite walls of the housing such that gas may circulate though the interior of the device thereby getting in touch with the chemical sensor arranged inside the housing between the two openings.

In another embodiment, the opening may be an opening already existing in the housing for a different purpose. In case of the device being embodied as a mobile phone or a voice controllable computing device, a small opening in the housing may be provided for a microphone. In an embodiment, which is not claimed, the chemical sensor may be arranged underneath / in such opening for the microphone together with the microphone. Another opening is provided for a speaker of the mobile phone. The chemical sensor may be arranged underneath / in such opening for the speaker together with the speaker. In an embodiment, which is not claimed, there may be a dedicated opening for the chemical sensor and an associate sensor if any. Such dedicated opening may be arranged in the housing elsewhere subject to different criteria such as space, connectivity, etc.

In another embodiment, there may be at least one additional sensor provided. Such at least one additional sensor may be a sensor / sensors out of the following list of sensors:
- a humidity sensor,
- a temperature sensor,
- a microphone,
- a pressure sensor,
- an airflow sensor.

Preferably, a temperature sensor and/or a humidity sensor are provided, as these sensor/s may help in compensating temperature induced and/or humidity induced signal variations in a signal of the chemical sensor. Preferably the temperature sensor and/or the humidity sensor may be arranged in proximity to the chemical sensor, for example in the same opening.

Irrespective of the kind of additional sensor applied to the system, such additional sensor may make use of the same opening as the chemical sensor does for getting access to the environment. Hence, such opening may be used by both or even more sensors if applicable.

The sensors may preferably be both arranged on the conductor board in close proximity. In another variant, the two or more sensors may be arranged on different conductor boards. In any case, in such combination, the chemical sensor may be an individual sensor also denoted as sensor cell, or may include an array of chemical sensor cells such as described above. As a further alternative, the conductor board may carry the additional sensor and one or more chemical sensor arrays and/or one or more individual chemical sensors of a discrete type.

In another embodiment, two or more openings may be provided in the housing for granting access to the sensors inside wherein one opening may be assigned to one of the sensors.

In another preferred embodiment, in which at least one additional sensor is used, the at least two sensors may be arranged in a common encapsulation, for example in a common mould compound encapsulating the two or more sensor chips the sensor structures are integrated in. In case two or more integrated sensor chips are used, these chips may be arranged on a lead frame or a different intermediary electric carrier, and subsequently may be encapsulated by a common encapsulation with at least connect pads remaining accessible in such encapsulation. The encapsulation may be formed such that sensitive structures of the sensor chips are not covered by the encapsulation either. Such package may then be bonded to the conductor board, and preferably may be assigned to a common opening in the housing wall. Especially, the chemical sensor and a humidity sensor may be arranged on such intermediary carrier and may be encapsulated together. The result is a compact package which package may be arranged even in small openings. An access opening in the encapsulation may be common to the chemical sensor and the humidity sensor, or separate access openings may be provided in the encapsulation. Other sensor chips such as a microphone may be added to such package, too, if desired. In any case, in any of the above embodiments, the package may contain a chemical sensor in form of an individual sensor cell of a discrete type, or may include an array of chemical sensor cells monolithically integrated on a common substrate. As a further alternative, the package may contain the additional sensor and one or more monolithically integrated chemical sensor arrays and/or one or more individual chemical sensor of a discrete type. In case a seal is provided, a single seal ring, for example, may be applied encircling the sensor structures of both the chemical sensor and the additional sensor. Such seal ring may be applied between the package and the housing wall.

In another embodiment, the chemical sensor and at least one additional sensor may be monolithically integrated in a common chip. Such chip includes a common substrate, on/in which the at least two different sensor structures representing at least two different sensor functions are integrated. Preferably, the sensors may be the chemical sensor and a humidity sensor, however other combinations such as the chemical sensor and a temperature sensor and/or a microphone may be envisaged, too. In such example, the humidity sensor may include a polymer layer deposited on the semiconductor substrate. The chemical sensor may include at least one more polymer layer susceptible to the multiple analytes or multiple different layers each susceptible to a designated analyte. In another embodiment, the chemical sensor may comprise a thinned substrate portion in combination with one or more metaloxide layers. After having manufactured such monolithic sensor chip in which different sensor functions are integrated, the resulting chip may be packaged, too, i.e. preferably cast into a mould compound which resulting package may then, for example, be SMD mounted to the conductor board. In any case, in any of the above embodiments, the monolithically integrated chip may contain a chemical sensor in form of an individual sensor cell, or may include an array of chemical sensor cells, or a combination of, in addition to the monolithically integrated additional sensor. In case a seal is provided, a single seal ring, for example, may be applied encircling the sensor structures of both the chemical sensor and the additional sensor. Such seal ring may be applied between the package if present and the housing wall or between the chip and the housing wall.

In a very specific embodiment, the conductor board may be formed by at least a part of a wall of the housing, and preferably by the wall that comprises the opening. Conductors may be applied to the inside surface of such wall, and electronic elements including the sensor chip containing the chemical sensor and possibly other sensors may directly mechanically and electrically be connected to the wall of the housing thereby electrically connecting the conductors on the wall. Specifically, the chemical sensor may be arranged across the opening with its sensitive structure facing the opening.

Other advantageous embodiments are listed in the dependent claims as well as in the description below.

### Brief Description of the Drawings

The embodiments defined above and further aspects, features and advantages of the present invention can also be derived from the examples of embodiments to be described hereinafter and are explained with reference to the drawings. In the drawings the figures illustrate in
FIG. 1 a usage scenario with a mobile phone according to an embodiment outside the scope of the present invention,
FIG. 2 a block diagram of a portable electronic device according to an embodiment of the present invention, and
FIG. 3 - 10 partial cuts through portable electronic devices according to various embodiments of the present invention.
FIG. 11 a cut through a chemical sensor package according to an embodiment of the present invention,
FIG. 12 a cut through a chemical sensor package according to another embodiment of the present invention, and
FIG. 13 a top view on a chemical sensor chip according to an embodiment of the present invention.

### Modes for Carrying Out the Invention

Same or similar elements are referred to by the same reference numerals across all Figures.

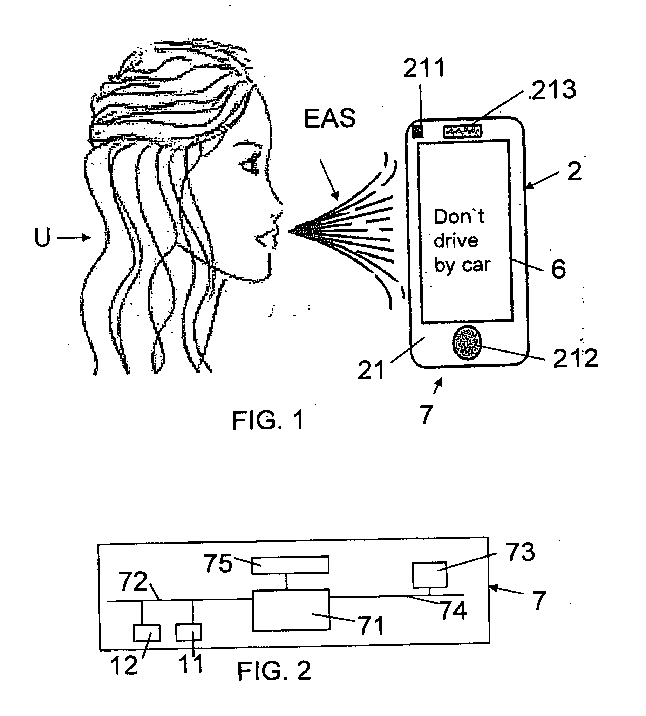

Figure 1 illustrates a usage scenario with a mobile phone 7 according to an embodiment outside the scope of the present invention. Apart from a standard microphone as an input device which microphone is arranged in an opening 212 of a front wall 21 of a housing 2 - which microphone may also be arranged in an opening of a side wall of the mobile phone 7 -, in an arrangement, which is not claimed, a chemical sensor is arranged in another opening 211 of the front wall 21, which opening 211 is arranged in proximity to yet another opening 213 for a standard speaker of the mobile phone 7. A user U blows at the mobile phone 3 such that at least a part of an exhalation air stream EAS meets the chemical sensor in the opening 211. A chemical sensor signal as response to the exhalation air stream is analyzed in the following. Specifically, the chemical sensor being sensitive to ethanol as one out of multiple analytes may sense a concentration of ethanol in the sample air stream. In case the analysis shows that the ethanol concentration exceeds a given threshold, a message may be displayed on a display 6 of the mobile phone, such as "Don't drive by car".

Figure 2 shows a schematic hardware oriented block diagram of a portable electronic device 7. Here, a microprocessor 71 is connected via a electrical conductors 72 to multiple sensors including the chemical sensor 12, and a microphone 11, for example, which electrical conductors 72 specifically may be conductors of a flexible printed circuit board. A routine for analysing the signals supplied by the sensors 12 and 11 may be executed on the microprocessor 71, which routine is stored in a memory 73 connected to the microprocessor 71 via a bus system 74. A wireless interface 75 is connected to the microprocessor 71, too. Some or all of the components of Figure 2, and specifically the sensors 12 and 11 and the bus system 72 may be arranged on a flexible printed circuit board forming a conductor board. The microprocessor 71, the memory 73 and the bus system 72 may be arranged there, too.

Prior to further embodiments, it is noted that the chemical sensor 12 may generally take different appearances in terms of packaging:
In a first embodiment, the chemical sensor may comprise a chip, preferably comprising a semiconductor substrate containing the sensitive structure/s on its top surface for example. Through-silicon-vias may provide an electrical connection between the top surface and a back side of the chip. At the back side of the chip, the through-silicon-vias may lead into contact pads which are provided with solder bumps. The chemical sensor 12 may then be pressed against a conductor board to be mounted to (SMD mounting), possibly under impact of heat, such that an electrical connection as well as a mechanical fixture is built between the chemical sensor and the conductor board. This variant of a chemical sensor package' in the following is also denoted as TSV-variant (for through-silicon-vias).

In a different embodiment, the chip is first mounted to an intermediate carrier such as a leadframe where the chip may be bonded to. Then the chip and the leadframe may be encapsulated in a moulding process, preferably with the sensitive structure being relieved from the encapsulation such that an access opening is formed in the encapsulation, which access opening may be referred to as 121 in the drawings. Contact pads on the lead frame may also remain free from encapsulation material. Such contact pads may also be provided with solder bumps. This variant of a chemical sensor package in the following is also denoted as mould-variant.

In a third variant, the chip as such with the sensitive structure and contact pads on the same surface is applied to a conductor board with the sensitive structure facing the conductor board. This variant of a chemical sensor in the following is also denoted as a flip-chip-variant. It is noted that in all of the following embodiments, a seal - although not shown - may preferably be applied between the chemical sensor and the housing, or between the conductor board and the housing if applicable. The seal in the first instance encloses the opening in the housing and the sensitive structure while in the second instance it encloses the opening in the housing and the chemical sensor as such.

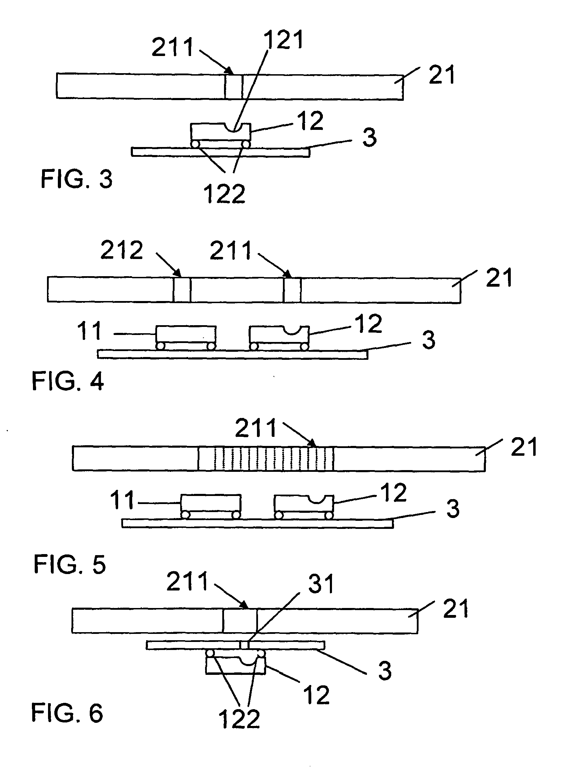

Figure 3 schematically illustrates a partial cut through a portable electronic device such as a tablet computer, for example. From an entire housing 2 only a part of the upper wall 21 / front wall 21 is shown which front wall 21 comprises an opening 211. Inside the housing, a printed circuit board 3 - which specifically may in an alternative be a flexible printed circuit board - is arranged more or less in parallel to the front wall 21, which printed circuit board 3 supports a chemical sensor 12. The chemical sensor 12 may be embodied in the mould-variant or in the TSV-variant and is attached to the printed circuit board 3 via solder balls 122.

The chemical sensor 12 is arranged in close proximity underneath the opening 211 in the front wall 21 of the housing such that the sensitive structure is exposed to any gas in the environment of the tablet computer that may diffuse into the interior of the housing through the opening 211. The printed circuit board 3 may be fixed elsewhere within the housing, and preferably may carry a processing unit for analyzing the signal supplied by the chemical sensor 12. The chip representing the chemical sensor 12 may additional hold integrated electronic circuitry. In a very preferred embodiment, the electronic circuitry and the sensitive structure may both be realized by the very same manufacturing process such as a CMOS process and possibly other additional processes such as MEMS processes. In such scenario, the chemical sensor chip may already provide a linearized digital signal, which may be supplied via conductors on the printed circuit board 3 to the processing unit.

Figure 4 illustrates a partial cut through a portable electronic device according to a different embodiment of the present invention. Such device is different to the portable electronic device of Figure 3 in that an additional sensor is arranged on the printed circuit board 3 - which specifically may in an alternative be a flexible printed circuit board. The additional sensor may preferably be a humidity sensor 11 for measuring a relative humidity of the air provided which constitutes a preferred combination of sensors when the portable electronic device is a mobile phone, for example. In addition, a temperature sensor may be added to the humidity sensor. However, in an embodiment, which is not claimed, the other sensor may also be a microphone, for example. The humidity sensor 11 and the chemical sensor 12 are arranged in close proximity on the same printed circuit board 3. Since both sensors 11 and 12 do have to rely on an access to the environment of the device, an additional opening 212 is provided in the front wall 21 of the housing. By such arrangement, which is not claimed, and in case the chemical sensor 12 is sensitive to ethanol and the additional sensor being a microphone, for example, a

measurement as to the alcohol status of user can be taken once the user speaks into the microphone 11 during a call, for example.

Figure 5 illustrates another cut through a portable electronic device according to another embodiment of the present invention. The present device is different to the device of Figure 4 in that both sensors 11 and 12 are assigned a common opening 211 in the front wall 21 of the housing. Such common opening 211 may alternatively be built from multiple small bores in the front wall 21 such as indicated by the dotted lines.

For the embodiments according to Fig. 4 and Fig. 5, the chemical sensor as well as the humidity sensor 11 may be embodied in one of the TSV-variant and the mould-variant.

Figure 6 illustrates a cut through a different portable device according to an embodiment of the present invention based on a different arrangement of the chemical sensor 12 and the printed circuit board 3. Here, the printed circuit board 3 - which specifically may in an alternative be a flexible printed circuit board - is arranged between the chemical sensor 12 and the front wall 21 while in all the previous embodiments, the chemical sensor 12 is arranged between the printed circuit board 3 and the front wall 21. In the present context, however, the chemical sensor 12 may be mounted to a backside of the printed circuit board 3. The preferred variant of the chemical sensor in this context is the flip-chip variant. Hence, the chemical sensor 12 is arranged such that its sensitive structure faces the printed circuit board 3. In order to provide better access to fluids entering the interior of the housing by means of the opening 211, the printed circuit board 3 also provides for an opening 31 which preferably is aligned with the sensitive structure of the chemical sensor 12. Preferably, the printed circuit board 3 is arranged such that the opening 211 in the front wall 21 and the opening 31 in the printed circuit board 3 are aligned, too, i.e. at least overlap.

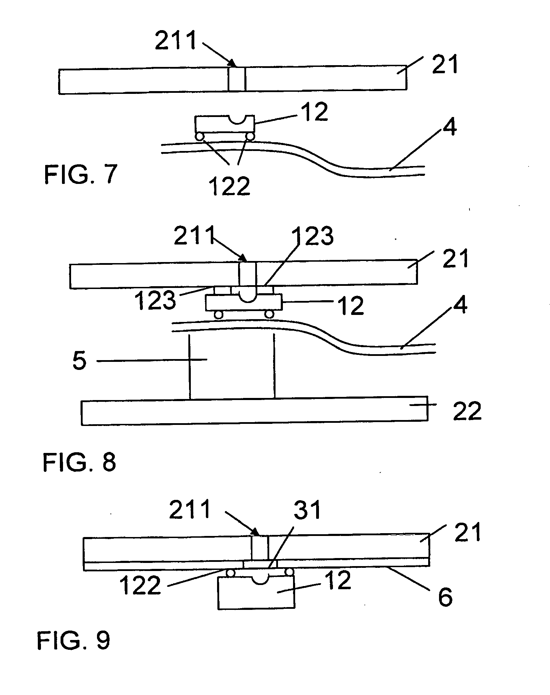

The embodiment of Figure 7 differs from the embodiment of Figure 3 in that instead of a rigid printed circuit board a flexible printed circuit board 4 is used. Such flexible printed circuit board 4 may offer an improved usage of space within the interior of the housing since it can be bent and may better align to any shape in the interior of the housing. A flexible printed circuit board may be made of a plastic material, such as Du-Pont's® Kapton® polyimide film of some 10 to some 100 µm thickness. Alternative materials are e.g. PET or LCP. The flexible printed circuit board 4 is flexible in the sense that it can be bent to a radius 2 cm or less, in particular 1 cm or less, without being damaged.

Figure 8 differs from Figure 7 in that it illustrates how the flexible printed circuit board may be fixed within the housing. A support element 5 is provided which on the one hand sits on a bottom wall 22 of the housing. At its opposite end the support element 5 supports the flexible printed circuit board 4, especially in a region where the chemical sensor 12 is arranged. On the other hand, a seal ring 123 is arranged on top of the chemical sensor chip and specifically surrounds the sensitive structure, i.e. either sits on the chip surface itself in case of the TSV variant, or sits on the encapsulation in case of the mould-variant. This seal ring 123 may be of compressible nature. In such embodiment, the chemical sensor 12 may be held in its position by clamped housing halves represented by the front wall 12 and the bottom wall 22. When clamping these walls 21 and 22 the seal ring 123 may be compressed and as a result fixes the chemical 12 sensor in its position. In addition, the opening 211 may be sealed by the seal ring 123 against the interior of the housing such that the device may become gas- and/or water-tight.

In an alternative, the support element 5 may be of elastic nature, too, and may generate a force acting on the conductor board for holding it is a defined position. Of course, both, an elastic support element 5 and an elastic seal ring 123 may be used in combination.

Figure 9 illustrates a different embodiment of a portable electronic device, now with a different embodiment of a conductor board. In the present example, either conductors or a conductor foil 6 may be applied, and specifically may be bonded to an inside surface of the front wall 21 containing the opening 211. Hence, the housing itself, and specifically the front wall 21 that holds the opening 211 may be interpreted as conductor board. The chemical sensor 12 now is soldered with its front side to the conductor foil 6, hence, the arrangement of and the necessities with respect to the chemical sensor 12 resemble the one of Figure 6. The flip-chip variant of the chemical sensor 12 preferably faces the opening 211 of the front wall 21. In addition, the conducting foil 6 provides an opening 31 facing the sensitive structure of the chemical sensor 12, too, which opening 31 is aligned with the opening 211 in the front wall 21. Such arrangement may save a lot of space in the interior of the housing. In this embodiment, the solder balls 122 provide for electrical connections to the conducting foil 6 similar to Fig. 6.

In such embodiment, a seal may preferably be applied by casting a seal material between the conductor foil 6 and the front side of the chemical sensor chip without covering the sensitive structure of the chemical sensor chip.

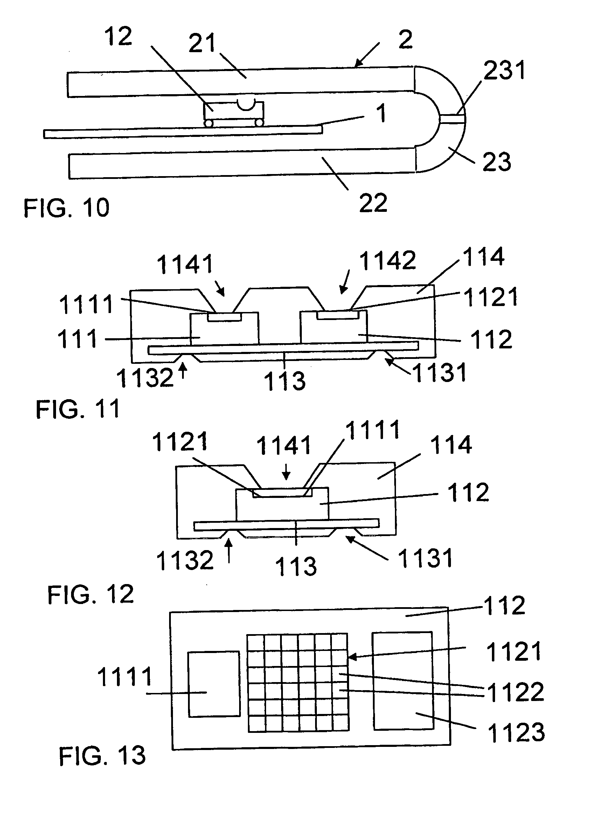

In Figure 10, a different embodiment of a housing is illustrated in a partial cut. The housing 2 contains a chemical sensor 12 inside. The chemical sensor 12 may be arranged on a printed circuit board 1 comparable to Figure 3. The housing 2 provides for a front wall 21, a bottom wall 22 and a side wall 23 defining the housing 2. Now, the opening no longer is provided in the front wall 21 close to the chemical sensor 12 but is arranged in the side wall 23 in form of opening 231. Such arrangement may be sufficient for granting access of environmental gas to the chemical sensor 12 while in some scenarios, it may be preferred for design or technical considerations to have the opening 231 arranged in the side wall 23.

Figure 11 illustrates a cut through a package containing a humidity sensor chip 111 and a chemical sensor chip 121 arranged on a common intermediary carrier such as a lead frame 113 which chips 111 and 112 are encapsulated by a common encapsulation 114, such as by an epoxy mould compound, hence forming a common package for both of the sensors. The chips 111 and 112 may be wire bonded to the lead frame (not shown). Each chip 111 and 112 comprises a sensitive structure 1111 and 1121. Recesses 1141 and 1142 in a top surface of the encapsulation 114 provide access to the sensitive structures 1111 and 1121. On a bottom surface of the encapsulation 114, access is provided to contact pads 1131 and 1132 formed by the lead frame 113. Such package may be arranged on the conductor board as shown in any one of the preceding embodiments.

Figure 12 illustrates a cut through a package containing a chemical sensor chip 112 in which in addition to the chemical sensitive structure 1121 another sensitive structure 1111 such as a humidity sensitive structure is monolithically integrated. Hence the single chip 112 carries multiple sensitive structures 1111, 1121 arranged in/on a common substrate. Hence, only a single recess 1141 may be required in the encapsulation 114. Such package may be arranged on the conductor board as shown in any one of the preceding embodiments.

Figure 13 illustrates a top view of a chemical sensor chip 112 such as may be used, for example, in the package of Figure 12. The chemical sensor structure 1121 takes the shape of a sensor array comprising multiple sensor cells 1122, in the present example, thirty six sensor cells 1122. In addition the humidity sensitive structure 1111 is arranged next to the chemical sensor array, and electronic circuitry 1123 is integrated into the chemical sensor chip 112, too.

While there are shown and described presently preferred embodiments of the invention, it is to be distinctly understood that the invention is not limited thereto but may be otherwise variously embodied and practised within the scope of the following claims.

## Claims

1. Portable electronic device, comprising
a housing (2),
a chemical sensor (12) arranged inside the housing (2), which chemical sensor (12) is adapted to measure a property of different analytes, and
an opening (211) in the housing (2) for exposing the chemical sensor (12) to a fluid to be analysed,
wherein the opening (211) is an opening (213) in the housing (2) also used for arranging a speaker.

2. Portable electronic device according to claim 1, wherein the chemical sensor (12) comprises an array of sensor cells (1122).

3. Portable electronic device according to claim 1 or claim 2,
wherein the chemical sensor (12) is arranged on a conductor board, and
wherein the chemical sensor (12) is connected via the conductor board to a processing unit (71).

4. Portable electronic device according to claim 3, wherein the chemical sensor (12) is arranged between the conductor board and a wall of the housing (2) comprising the opening (211), and wherein the chemical sensor (12) faces the opening (211).

5. Portable electronic device according to claim 3, wherein the opening (211) is arranged in a side wall (23) of the housing (2), and wherein the side wall (23) faces a front end of the conductor board arranged in the interior of the housing (2).

6. Portable electronic device according to claim 3,
wherein the conductor board is arranged between the chemical sensor (12) and a wall of the housing (2) comprising the opening (211),
wherein the chemical sensor (12) is arranged on the conductor board with a sensitive element (1121) of the chemical sensor (12) facing the conductor board,
wherein the conductor board comprises an opening (31) for connecting the chemical sensor (12) to the opening (211) in the housing (2), and
wherein the opening (31) in the conductor board is aligned with the opening (211) in the housing (2).

7. Portable electronic device according to any one of the preceding claims, comprising at least one additional sensor out of the following list of sensors:
- a humidity sensor,
- a temperature sensor,
- a microphone,
- a pressure sensor,
- an airflow sensor.

8. Portable electronic device according to claim 7,
comprising the temperature sensor for compensating temperature dependent signal variations in a signal of the chemical sensor (12), and/or
comprising the humidity sensor for compensating humidity dependent signal variations in a signal of the chemical sensor (12).

9. Portable electronic device according to claim 7 or claim 8, wherein the chemical sensor (12) and the at least one additional sensor are arranged on a common conductor board.

10. Portable electronic device according to any one of the preceding claims 7 to 9, wherein the chemical sensor (12) and the at least one additional sensor comprise a common encapsulation.

11. Portable electronic device according to any one of the preceding claims 7 to 10,
wherein the chemical sensor (12) and the at least one additional sensor are integrated in a common chip (112) comprising a common semiconductor substrate, and
wherein the common chip (112) additionally comprises electronic circuitry (1123) connected to the chemical sensor (12) and to the at least one additional sensor.

12. Portable electronic device according to any one of the preceding claims 7 to 11, wherein the chemical sensor (12) and the at least one additional sensor face the same common opening (211).

13. Portable electronic device according to any one of the preceding claims 7 to 12, wherein one or more different openings (212) are assigned to the at least one additional sensor.

14. Portable electronic device according to any one of the preceding claims, wherein the chemical sensor (12) is arranged in the housing (2) between two openings of opposite walls of the housing (2).

15. Portable electronic device according to claim 9,
wherein the conductor board is a flexible printed circuit board (3).

16. Portable electronic device according to claim 9,
wherein the conductor board is formed by a wall of the housing (2) carrying the opening (211) by having applied conducting structures (6) on an inner surface of the wall, and
wherein the chemical sensor (12) is arranged across the opening (211) with a sensitive element of the chemical sensor (12) facing the opening (211).

17. Portable electronic device according to claim 9, comprising a support element (5) for supporting the conductor board, and
wherein the support element is of elastic property and wherein at least the elastic element (5) and the conductor board are clamped between two opposing walls of the housing (2).

18. Portable electronic device according to any one of the preceding claims, wherein a seal (123) is arranged between the chemical sensor (12) and the housing (2) for sealing the opening (211) against an interior of the housing (2).

19. Portable electronic device according to any one of the preceding claims, wherein the opening (211) is covered by one or more of: a membrane permeable to analytes of interest, a grid, slits and a bezel.

20. Portable electronic device according to any one of the preceding claims,
which portable electronic device is one of:
a mobile phone,
a handheld computer,
an electronic reader,
a tablet computer,
a game controller,
a pointing device,
a photo or a video camera,
a computer peripheral.

## Patentansprüche

1. Tragbares elektronisches Gerät, umfassend
ein Gehäuse (2),
einen Chemie-Sensor (12), welcher im Gehäuse (2) angeordnet ist, wobei der Chemie-Sensor (12) dazu geeignet ist, eine Eigenschaft von verschiedenen Analyten zu Messen und
eine Öffnung (211) im Gehäuse (2), um den Chemie-Sensor (12) einem Fluid, welches analysiert werden soll, auszusetzten,
wobei die Öffnung (211) eine Öffnung (213) im Gehäuse (2) ist, welche auch dafür benutzt wird, einen Lautsprecher anzuordnen.

2. Tragbares elektronisches Gerät nach Anspruch 1, wobei der Chemie-Sensor (12) eine Anordnung von Sensorzellen (1122) enthält.

3. Tragbares elektronisches Gerät nach einem der Ansprüche 1 oder 2,
wobei der Chemie-Sensor (12) auf einer Leiterplatte angeordnet ist und
wobei der Chemie-Sensor (12) über die Leiterplatte mit einer Steuereinheit (71) verbunden ist.

4. Tragbares elektronisches Gerät nach Anspruch 3, wobei der Chemie-Sensor (12) zwischen der Leiterlatte und einer Wand des Gehäuses (2) angeordnet ist, welche die Öffnung (211) enthält, und wobei der Chemie-Sensor (12) der Öffnung (211) zugewandt ist.

5. Tragbares elektronisches Gerät nach Anspruch 3, wobei die Öffnung (211) in einer Seitenwand (23) des Gehäuses (2) angeordnet ist und wobei die Seitenwand (23) einer Stirnseite der Leiterplatte im Innern des Gehäuses (2) zugewandt ist.

6. Tragbares elektronisches Gerät nach Anspruch 3,
wobei die Leiterplatte zwischen dem Chemie-Sensor (12) und einer Wand des Gehäuses (2), welche die Öffnung (211) enthält, angeordnet ist,
wobei der Chemie-Sensor (12) so auf der Leiterplatte angeordnet ist, dass ein sensitives Element (1121) des Chemie-Sensors (12) der Leiterplatte zugewandt ist,
wobei die Leiterplatte eine Öffnung (31) enthält, um den Chemie-Sensor (12) mit der Öffnung (211) im Gehäuse (2) zu verbinden, und
wobei die Öffnung (31) in der Leiterplatte und die Öffnung (211) im Gehäuse zueinander ausgerichtet sind.

7. Tragbares elektronisches Gerät nach einem der vorhergehenden Ansprüche, mindestens einen zusätzlichen Sensor aus der folgenden Liste von Sensoren enthaltend:
- ein Feuchtigkeitssensor,
- ein Temperatursensor,
- ein Mikrofon,
- ein Drucksensor,
- ein Luftflusssensor.

8. Tragbares elektronisches Gerät nach Anspruch 7,
den Temperatursensor enthaltend, um von der Temperatur abhängige Signalschwankungen eines Signals des Chemie-Sensors (12) zu kompensieren, und/oder
den Feuchtigkeitssensor enthaltend, um von der Feuchtigkeit abhängige Signalschwankungen eines Signals des Chemie-Sensors (12) zu kompensieren.

9. Tragbares elektronisches Gerät nach einem der Ansprüche 7 oder 8, wobei der Chemie-Sensor (12) und mindestens ein zusätzlicher Sensor auf einer gemeinsamen Leiterplatte angeordnet sind.

10. Tragbares elektronisches Gerät nach einem der vorhergehenden Ansprüche 7 bis 9, wobei der Chemie-Sensor (12) und mindestens ein zusätzlicher Sensor eine gemeinsame Einkapselung haben.

11. Tragbares elektronisches Gerät nach einem der vorhergehenden Ansprüche 7 bis 10,
wobei der Chemie-Sensor (12) und mindestens ein zusätzlicher Sensor in einen gemeinsamen Chip (112) integriert sind, welcher ein gemeinsames Halbleitersubstrat umfasst, und
wobei der gemeinsame Chip (112) zusätzlich Elektronik (1123) enthält, welche mit dem Chemie-Sensor (12) und mindestens einem zusätzlichen Sensor verbunden ist.

12. Tragbares elektronisches Gerät nach einem der vorhergehenden Ansprüche 7 bis 11, wobei der Chemie-Sensor (12) und mindestens der eine zusätzlicher Sensor der gemeinsamen Öffnung (211) zugewandt sind.

13. Tragbares elektronisches Gerät nach einem der vorhergehenden Ansprüche 7 bis 12, wobei eine oder mehrere verschiedene Öffnungen (212) mindestens dem einen zusätzlichen Sensor zugeordnet sind.

14. Tragbares elektronisches Gerät nach einem der vorhergehenden Ansprüche, wobei der Chemie-Sensor (12) im Gehäuse (2) zwischen zwei Öffnungen gegenüberliegender Wände des Gehäuses (2) angeordnet ist.

15. Tragbares elektronisches Gerät nach Anspruch 9,
wobei die Leiterplatte eine flexible, gedruckte Leiterplatte (3) ist.

16. Tragbares elektronisches Gerät nach Anspruch 9,
wobei die Leiterplatte durch eine Wand des Gehäuses (2) gebildet wird, welche die Öffnung (211) enthält, wobei auf einer Innenseite der Wand des Gehäuses (2) leitende Strukturen (6) angebracht wurden und
wobei der Chemie-Sensor (12) über der Öffnung (211) angeordnet ist, so dass ein sensitives Element des Chemie-Sensors der Öffnung (211) zugewandt ist.

17. Tragbares elektronisches Gerät nach Anspruch 9, umfassend ein Stützelement (5), um die Leiterplatte zu stützen und
wobei das Stützelement elastische Eigenschaften hat und wobei mindestens das elastische Element (5) und die Leiterplatte zwischen zwei gegenüberliegende Wände des Gehäuses (2) geklemmt sind.

18. Tragbares elektronisches Gerät nach einem der vorhergehenden Ansprüche, wobei eine Dichtung (123) zwischen dem Chemie-Sensor (12) und dem Gehäuse (2) angeordnet ist, um die Öffnung (211) gegen ein Inneres des Gehäuses (2) abzudichten.

19. Tragbares elektronisches Gerät nach einem der vorhergehenden Ansprüche, wobei die Öffnung (211) abgedeckt ist durch eines oder mehrere von: eine Membran, durchlässig für die zu prüfenden Analyten, ein Gitternetz, Schlitze und eine Blende.

20. Tragbares elektronisches Gerät nach einem der vorhergehenden Ansprüche,
wobei das tragbare elektronische Gerät eines aus Folgenden ist:
ein Mobiltelefon,
ein tragbarer Computer,
ein elektronisches Lesegerät,
ein Tablet-Computer
ein Spielecontroller,
ein Zeigegerät,
eine Foto- oder Videokamera,
ein Computerperipheriegerät.

## Revendications

1. Dispositif électronique portable, comprenant
une carcasse (2),
un capteur chimique (12) arrangé à l'intérieur de la carcasse (2), le capteur chimique (12) étant adapté à mesurer une propriété des différents analytes, et
une ouverture (211) dans la carcasse (2) pour exposer le capteur chimique (12) à un fluide à être analysé,
l'ouverture (211) étant une ouverture (213) dans la carcasse (2) également utilisée pour loger un haut-parleur.

2. Dispositif électronique portable selon la revendication 1, le capteur chimique (12) comprenant un réseau de cellules de capteur (1122).

3. Dispositif électronique portable selon la revendication 1 ou la revendication 2, le capteur chimique (12) étant arrangé sur un circuit imprimé, et
le capteur chimique (12) étant connecté à une unité de traitement (71) par l'intermédiaire du circuit imprimé.

4. Dispositif électronique portable selon la revendication 3, le capteur chimique (12) étant arrangé entre le circuit imprimé et une paroi de la carcasse (2) comprenant l'ouverture (211), et le capteur chimique (12) étant tourné vers l'ouverture (211).

5. Dispositif électronique portable selon la revendication 3, l'ouverture (211) étant arrangée dans une paroi latérale (23) de la carcasse (2), et la paroi latérale (23) étant tournée vers une face frontale du circuit imprimé arrangé à l'intérieur de la carcasse (2).

6. Dispositif électronique portable selon la revendication 3, le circuit imprimé étant arrangé entre le capteur chimique (12) et une paroi de la carcasse (2) comprenant l'ouverture (211),
le capteur chimique (12) étant arrangé sur le circuit imprimé avec un élément sensible (1121) du capteur chimique (12) tourné vers le circuit imprimé,
le circuit imprimé comprenant une ouverture (31) pour connecter le capteur chimique (12) avec l'ouverture (211) dans la carcasse (2), et
l'ouverture (31) dans le circuit imprimé étant alignée avec l'ouverture (211) dans la carcasse (2).

7. Dispositif électronique portable selon l'une des revendications précédentes, comprenant au moins un autre capteur de la liste suivante de capteurs:
- un capteur d'humidité,
- un capteur de température,
- un microphone,
- un capteur de pression,
- un capteur d'écoulement d'air.

8. Dispositif électronique portable selon la revendication 7,
comprenant le capteur de température pour compenser des variations de signal, dépendantes de la température, dans une signal du capteur chimique (12), et/ou
comprenant le capteur d'humidité pour compenser des variations de signal, dépendantes de l'humidité, dans une signal du capteur chimique (12).

9. Dispositif électronique portable selon la revendication 7 ou la revendication 8, le capteur chimique (12) et l'au moins un capteur additionnel étant arrangés sur un circuit imprimé commun.

10. Dispositif électronique portable selon l'une des revendications précédentes 7 à 9, le capteur chimique (12) et l'au moins un capteur additionnel comprenant une encapsulation commune.

11. Dispositif électronique portable selon l'une des revendications 7 à 10,
le capteur chimique (12) et l'au moins un capteur additionnel étant intégrés dans une puce commune (112) comprenant un substrat semi-conducteur commun, et
la puce commune (112) comprenant en outre des circuits électroniques (1123) connectés au capteur chimique (12) et à l'au moins un capteur additionnel.

12. Dispositif électronique portable selon l'une des revendications précédentes 7 à 11, le capteur chimique (12) et l'au moins un capteur additionnel étant tournés vers la même ouverture commune (211).

13. Dispositif électronique portable selon l'une des revendications précédentes 7 à 12, une ou plusieurs ouvertures (212) différentes étant attribuées à l'au moins un capteur additionnel.

14. Dispositif électronique portable selon l'une des revendications précédentes, le capteur chimique (12) étant arrangé dans la carcasse (2) entre deux ouvertures des parois différentes de la carcasse (2).

15. Dispositif électronique portable selon la revendication 9,
le circuit imprimé étant un circuit imprimé flexible (3).

16. Dispositif électronique portable selon la revendication 9,
le circuit imprimé étant formé par une paroi de la carcasse (2) portant l'ouverture (211) par des structures conductrices (6) y appliquées sur une surface intérieure de la paroi, et
le capteur chimique (12) étant arrangé à travers de l'ouverture (211) avec un élément sensible du capteur chimique (12) tourné vers l'ouverture (211).

17. Dispositif électronique portable selon la revendication 9, comprenant un élément de support (5) pour supporter le circuit imprimé, et
l'élément de support ayant une propriété élastique et au moins l'élément élastique (5) et le circuit imprimé étant serrés entre deux parois opposées de la carcasse (2).

18. Dispositif électronique portable selon l'une des revendications précédentes, un joint (123) étant arrangé entre le capteur chimique (12) et la carcasse (2) pour sceller l'ouverture (211) vers un espace intérieur de la carcasse (2).

19. Dispositif électronique portable selon l'une des revendications précédentes, l'ouverture (211) étant couverte par au moins une de: une membrane perméable aux analytes intéressants, une grille, des fentes et une lunette.

20. Dispositif électronique portable selon l'une des revendications précédentes,
le dispositif électronique portable étant: un des :
un téléphone portable,
un ordinateur de poche,
un lecteur électronique,
un ordinateur tablette,
un ordinateur du jeu,
un dispositif de pointage,
une caméra photo ou vidéo,
une périphérique d'ordinateur.
